# EUROPÄISCHE PATENTSCHRIFT

(11) **EP 1 562 509 B1**
(45) Veröffentlichungstag und Bekanntmachung des Hinweises auf die Patenterteilung: **22.02.2006**
(21) Anmeldenummer: 02777241.7
(22) Anmeldetag: 27.09.2002
(51) Int. Cl.: A61B 19/00, A61B 5/103

(54) **VORRICHTUNG ZUR BESTIMMUNG DER LAGE DES TIBIALEN AUSTRITTPUNKTES DES VORDEREN KREUZBANDES**
DEVICE FOR DETERMINING THE POSITION OF THE TIBIAL PROTRUSION POINT OF THE ANTERIOR CRUCIATE LIGAMENT
DISPOSITIF POUR DETERMINER LA POSITION DU POINT DE SORTIE TIBIAL DU LIGAMENT CROISE AVANT

(43) Veröffentlichungstag der Anmeldung: 17.08.2005
(73) Patentinhaber: Aesculap AG & Co. KG, 78532 Tuttlingen (DE)
(72) Erfinder: GIORDANO, Nicola, 78056 Villingen-Schwenningen (DE); LEITNER, François, F-38410 Uriage (FR); BOUX DE CASSON, François, F-38000 Grenoble (FR); EICHHORN, Jürgen, 94360 Mitterfels/Scheibelsgrub (DE)
(74) Vertreter: Hoeger, Stellrecht & Partner Patentanwälte
(86) Internationale Anmeldenummer: PCT/EP2002/010861
(87) Internationale Veröffentlichungsnummer: WO 2004/032780

(56) Entgegenhaltungen:
- EP-A- 0 603 089
- WO-A-99/23956
- US-A- 5 743 909
- US-A1- 2002 055 679

## Beschreibung

Die Erfindung betrifft eine Vorrichtung zur Bestimmung der Lage des Austrittspunktes eines das vordere Kreuzbandes eines Kniegelenkes ersetzenden Implantates an der Oberseite der Tibia mit einem Navigationssystem, mit fest mit dem Femur und der Tibia verbundenen Markierelementen, mit einem navigierten Taster und mit einer einen Speicher und eine Recheneinheit aufweisenden Datenverarbeitungsanlage, der die durch das Navigationssystem aufgenommenen Positionsdaten von mehreren Punkten längs der Vorderkante der Notch zuführbar und in den Speicher ablegbar sind.

Beim Ersetzen eines vorderen Kreuzbandes eines Kniegelenkes durch ein Implantat ist es außerordentlich wichtig, daß das Implantat exakt positioniert wird. Üblicherweise wird das Implantat an seinen beiden Enden in geeigneter Weise mit dem Femur bzw. der Tibia verbunden, beispielsweise durch Einführen in Bohrungen, die in Femur und Tibia eingebracht werden und deren Endpunkte an der Femuroberfläche und an der Tibiaoberfläche Austrittspunkte für das Implantat markieren.

Ein wichtiger Gesichtspunkt dabei ist der, daß die Länge des Implantates im Bereich zwischen den Austrittspunkten bei der Bewegung des Knies möglichst ungeändert bleiben soll, es soll also eine Isometrie des Implantates erreicht werden. Dazu ist es bekannt, den Austrittspunkt des Implantates am Femur durch ein Verfahren und eine Vorrichtung zu bestimmen, bei denen geometrische Daten der Femuroberfläche abgetastet und gespeichert werden und bei denen diese geometrischen Daten verwendet werden, um die Veränderung der Isometrie bei verschiedener Lage der Austrittspunkte zu berechnen (EP 603 089 B1). Mit diesem Verfahren ist es aber nur möglich, den femurseitigen Austrittspunkt in seiner Lage zu optimieren, bei der Auswahl des tibiaseitigen Austrittspunktes des Implantates kann dieses bekannte Verfahren keine Hilfestellung geben.

In der US 2002/0055679 A1 wird eine Vorrichtung beschrieben, mit der die Lage der Austrittspunkte bestimmt werden kann.

Ein wesentliches Problem bei der Bestimmung der Lage des tibiaseitigen Austrittspunktes liegt darin, daß das Implantat im eingesetzten Zustand bei allen Stellungen des Knies zwischen den beiden Austrittspunkten frei bleiben muß, es darf sich also nicht an Knochenstrukturen anlegen, beispielsweise an bestimmte Flächen des Femurs, da diese Anlage zu einer Schädigung des Implantates führen könnte. Das vordere Kreuzband und das sie ersetzende Implantat wird normalerweise in dem Zwischenraum zwischen den Gelenkflächen des Femurs, also den Kondylen, aufgenommen, diesen Zwischenraum bezeichnet man als die Notch. Diese Notch wird seitlich von den Seitenflächen der Kondylen begrenzt, an der Oberseite durch eine obere Dachfläche, und diese Dachfläche geht über eine markante Vorderkante an der Vorderseite des Femurs in die seitliche Außenfläche des Femurs über. Gerade diese Vorderkante kann bei unsachgemäßer Positionierung des Implantats des vorderen Kreuzbandes mit diesem Implantat in gewissen Stellungen des Beines kollidieren, und dies muß in jedem Fall vermieden werden.

Bisher hat der Operateur die Positionierung des tibiaseitigen Austrittspunktes des vorderen Kreuzbandes aufgrund seiner Erfahrung gewählt, dabei sind aber Fehlimplantationen nicht mit Sicherheit auszuschließen.

Es ist Aufgabe der Erfindung, eine Vorrichtung zur Verfügung zu stellen, mit der der Operateur beim Auffinden des tibiaseitigen Austrittspunktes so unterstützt wird, daß ein freier Verlauf des Implantates bei allen Beinstellungen gewährleistet ist.

Diese Aufgabe wird erfindungsgemäß durch eine Vorrichtung der eingangs beschriebenen Art gelöst, die dadurch gekennzeichnet ist, daß die Recheneinheit so programmiert ist, daß sie die in dem Speicher abgelegten Positionsdaten und damit den Verlauf der Vorderkante der Notch bei gestrecktem Bein auf eine Projektionsebene im Bereich der Tibiaoberseite projiziert, die senkrecht auf der Längsachse der Tibia steht, und damit eine U-förmige projizierte Kurve erzeugt, die einen Bereich umschließt, in dem sich der Austrittspunkt befindet. Dies ist ein von der Datenverarbeitungsanlage selbständig durchgeführter Vorgang, durch den ein Bereich einer Tibiaoberfläche definiert wird, der von der projizierten Kurve umgeben ist und in dem der gewünschte Austrittspunkt liegt.

Zur Bestimmung des tibiaseitigen Austrittspunktes wird also mittels der Datenverarbeitungsanlage folgendermaßen vorgegangen:
bei abgewinkeltem Bein erfaßt man die Position von mehreren Punkten längs der Vorderkante der Notch und bestimmt dadurch deren Lage und Verlauf am Femur,
bei gestrecktem Bein projiziert man den Verlauf dieser Vorderkante der Notch auf eine Projektionsebene, die im Bereich der Tibiaoberseite senkrecht auf der Längsachse der Tibia steht, und
innerhalb des von dieser projizierten Kurve umgebenen Bereichs wählt man den Austrittspunkt so aus, daß er von der projizierten Kurve mindestens einen Abstand einhält, der dem Radius des Implantates entspricht.

Immer dann, wenn der Austrittspunkt innerhalb des Bereichs der projizierten Kurve liegt, die im wesentlichen einen U-förmigen Verlauf einnimmt, ist sichergestellt, daß das Implantat bei allen Bewegungen des Knies nicht mit knöchernen Strukturen des Femurs kollidiert, insbesondere nicht mit der Vorderkante der Notch. Die Projektionsebene kann dabei in unterschiedlichem Abstand vom Femur ausgewählt werden, auf deren genaue Lage kommt es nicht an, sofern sich diese Ebene nur im oberen Tibiabereich befindet. Für den Operateur ist nur wichtig, in dieser Ebene eine geeignete Position des Austrittspunktes zu finden, und diese Information läßt sich auch erreichen, wenn die Ebene um einige Millimeter nach oben oder nach unten verschoben ist.

Dabei ist es vorteilhaft, wenn die Recheneinheit eine Kreisfläche, deren Durchmesser dem Durchmesser des verwendeten Implantates entspricht, in die Projektionsebene projiziert, diese in der von der projizierten Kurve umgebenen Fläche so verschiebt, daß sie die projizierte Kurve nicht schneidet, und wenn man in einer der so erreichten Positionen der Kreisfläche deren Mittelpunkt als Austrittspunkt des Implantates wählt.

Insbesondere kann vorgesehen sein, daß die Recheneinheit eine Lage des Austrittspunktes wählt, bei der dieser möglichst weit im vorderen Teil des von der projizierten Kurve umgebenen Bereichs und möglichst nahe an der projizierten Kurve liegt.

Bei einer bevorzugten Ausführungsform bildet die Datenverarbeitungsanlage bei der Erfassung der Position von Punkten auf der Vorderkante der Notch auf einer Bildanzeige die Vorderkante ab und markiert dort die Punkte, deren Positionsdaten zu erfassen sind.

Es ist günstig, wenn die Recheneinheit die projizierte Kurve und den von ihr umgebenen Bereich auf einer Bildanzeige darstellt, auf dieser Bildanzeige weiterhin ein verschiebbares Bild eines Anzeigeelementes für die ausgewählte Position des Austrittspunktes abbildet und die entsprechenden Positionsdaten der ausgewählten Position in einem Speicher ablegt. Insbesondere kann das Anzeigeelement die Form eines Kreises haben, dessen Durchmesser dem des verwendeten Implantates entspricht.

Die nachfolgende Beschreibung bevorzugter Ausführungsformen der Erfindung dient im Zusammenhang mit der Zeichnung der näheren Erläuterung. Es zeigen:
- Figur 1:: eine schematische Ansicht eines abgewinkelten Kniegelenks mit einem Navigationssystem zur Positionsbestimmung des Femurs, der Tibia und eines Tastelementes;
- Figur 2:: eine schematische Schnittansicht durch ein Kniegelenk in abgewinkelter Stellung;
- Figur 3:: eine schematische Vorderansicht des abgewinkelten Kniegelenks der Figur 2;
- Figur 4:: eine Ansicht ähnlich Figur 2 bei gestrecktem Kniegelenk und
- Figur 5:: eine Schnittansicht längs Linie 5-5 in Figur 4.

In der Zeichnung sind das distale Ende eines Femurs 1 und das proximale Ende einer Tibia 2 dargestellt, die gemeinsam ein Kniegelenk 3 ausbilden, die übrigen Teile des Kniegelenkes sind zur Verdeutlichung weggelassen. Der Femur 1 bildet in diesem Bereich zwei nebeneinander liegende Gelenkflächen aus, die Kondylen 4, 5, die zwischen sich einen Hohlraum aufnehmen. Diesen Hohlraum bezeichnet man üblicherweise als die Notch 6. Diese wird seitlich begrenzt von Seitenflächen 7 der Kondylen 4, 5. An der Oberseite erstreckt sich ein schräg zur Längsachse des Femurs 1 verlaufendes Dach 8. An der Vorderseite, also anterior, ist die Notch 6 geschlossen, zur Rückseite des Kniegelenks hin, also posterior, dagegen offen. Zu den Gelenkflächen der Kondylen 4, 5 hin wird die Notch 6 durch eine annähernd U-förmige Vorderkante 9 begrenzt. Diese bildet den Übergang zwischen einem knöchernen und einem knorpeligen Bereich und ist für den Operateur gut zu tasten und daher aufzufinden.

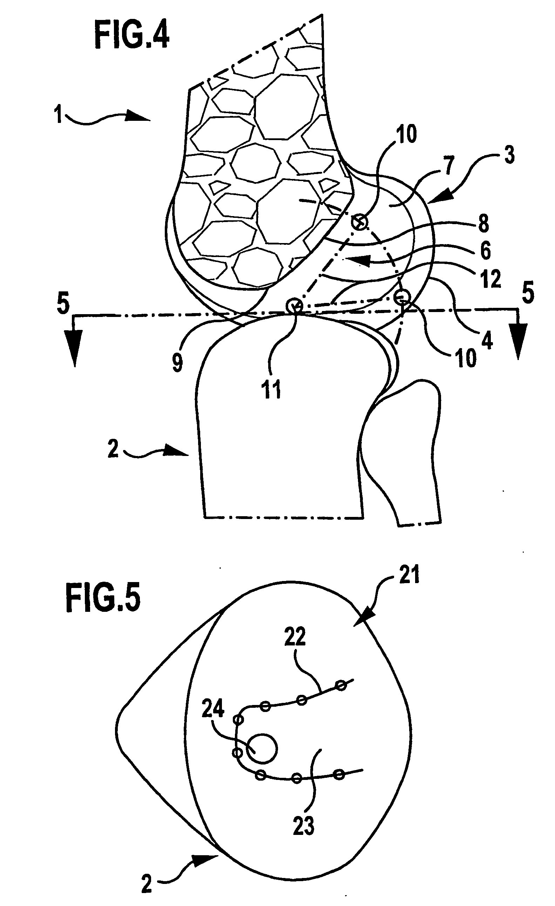

Femur 1 und Tibia 2 werden durch mehrere Bänder miteinander verbunden. Im vorliegenden Fall interessiert nur das sogenannte vordere Kreuzband, welches sich in dem Zwischenkondylenhohlraum, also in der Notch 6, erstreckt und femurseitig an einem am posterioren Ende der Notch 6 angeordneten Punkt 10 mit dem Femur 1 verbunden ist, tibiaseitig an einem im folgenden als Austrittspunkt 11 bezeichneten Punkt, der gegenüber dem Punkt 10 anterior versetzt ist. In Figur 4 sind der Punkt 10 und der Austrittspunkt 11 schematisch dargestellt, die Verbindungslinie 12 des Punktes 10 und des Austrittspunktes 11 zeigt den Verlauf des vorderen Kreuzbandes bei gestrecktem Knie (Punkt 10 in der hohen Position) und bei gebeugtem Knie (Punkt 10 in der abgesenkten Position).

Um das vordere Kreuzband durch ein Implantat zu ersetzen, welches beispielsweise ein künstliches Implantat sein kann oder ein Bandstück, das an anderer Stelle des Körpers entnommen worden ist, ist es notwendig, die Lage sowohl des Punktes 10 als auch des Austrittspunktes 11 zu bestimmen. Die Lage des Punktes 10 bestimmt sich im wesentlichen danach, daß man bei der Bewegung die Isometrie erhält, also eine möglichst geringe Längenänderung des Implantates. Bei der Lage des Austrittspunktes 11 muß dafür Sorge getragen werden, daß das Implantat nach der Implantation nicht mit der knöchernen Struktur des Femurs 1 in Kontakt kommt, insbesondere nicht mit der Vorderkante 9.

Um dies zu erreichen, werden an Femur 1 und Tibia 2 Markierelemente 13, 14 festgelegt, die beispielsweise drei Infrarotstrahlung reflektierende Kugeln aufweisen und die jeweils starr mit dem Femur bzw. der Tibia verbunden sind. Derartige Markierelemente 13 und 14 sind Teil eines an sich bekannten Navigationssystems 15, welches über ein Kamerasystem mit drei Kameras 16 von den Markierelementen reflektierte Strahlung auffängt und daraus die Lage und den Ort der Markierelemente 13 und 14 im Raum bestimmt und damit natürlich auch Lage und Ort des Femurs 1 und der Tibia 2. Die entsprechenden Positionsdaten werden im Navigationssystem 15 einer Datenverarbeitungsanlage 17 zugeführt und können dort gespeichert und zu nachfolgenden Berechnungen verwendet werden.

Zur Feststellung der Lage der Vorderkante 9 in dem Kniegelenk 3 wird weiter ein Tastelement 18 verwendet, beispielsweise ein einfacher Tasthaken, an dem ein weiteres Markierelement 19 gleicher Bauart befestigt ist, dessen Lage ebenfalls durch das Navigationssystem 15 bestimmt werden kann. Es ist dadurch möglich, Ort und Lage des Tastelements 18 jederzeit genau festzustellen.

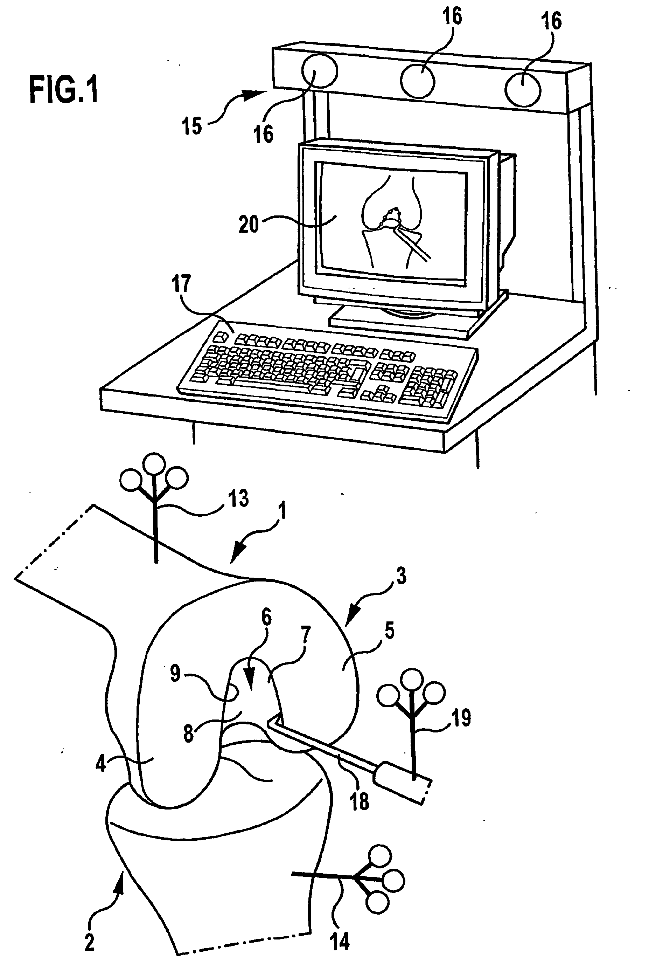

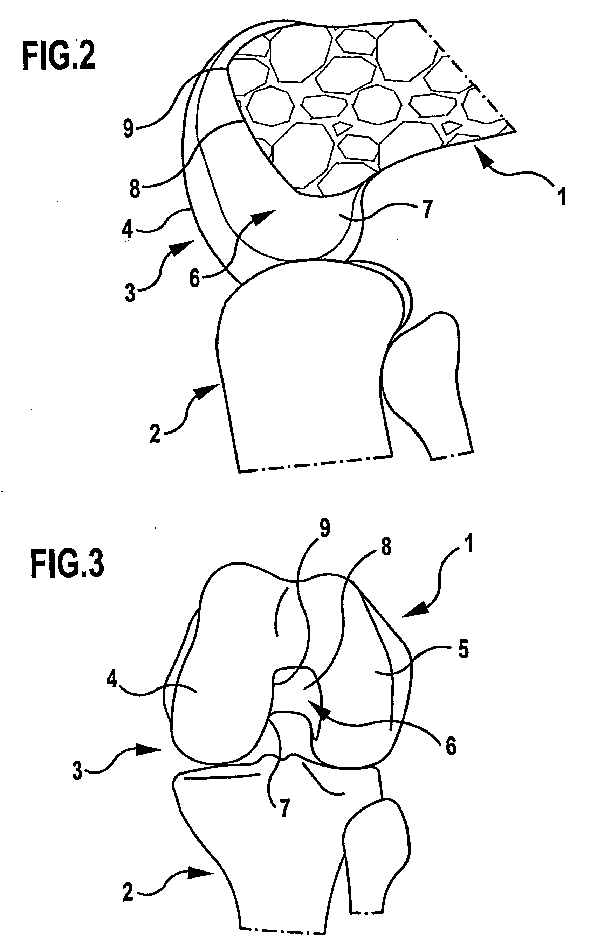

Zur Festlegung des tibiaseitigen Austrittspunktes 11 wird zunächst das Kniegelenk 3 so abgewinkelt, daß die Vorderkante 9 der Notch 6 frei zugänglich wird, dies ist in den Figuren 1 bis 3 dargestellt. Mit Hilfe des Tastelementes 18 werden eine größere Anzahl von einzelnen Punkten der Vorderkante angefahren und deren Positionsdaten in der Datenverarbeitungsanlage 17 gespeichert. Um dem Operateur diesen Vorgang zu erleichtern, kann auf einem Bildschirm 20 der Datenverarbeitungsanlage 17 schematisch der Verlauf einer solchen Vorderkante dargestellt werden. Auf dieser Vorderkante werden einzelne Punkte markiert, die dem Operateur zeigen, an welchen Stellen das Tastelement 18 an die Vorderkante 9 angelegt werden soll. Wenn an einer solchen Stelle Positionsdaten bestimmt und gespeichert worden sind, kann dies dem Operateur am Bildschirm 20 signalisiert werden, beispielsweise durch eine Farbänderung der entsprechenden Punkte, so daß der Operateur beim Abtasten der Vorderkante 9 so geführt wird, daß er über die gesamte Länge dieser Vorderkante gleichmäßig Meßpunkte anfährt und die Positionsdaten bestimmt. Diese Positionsdaten werden gemeinsam im Speicher abgelegt und beschreiben den Verlauf der Vorderkante am Femur 1.

In einem weiteren Schritt wird das Bein in die Streckstellung verschwenkt, wie dies in Figur 4 dargestellt ist. Durch die Markierelemente 13 und 14 werden dabei die Bewegungen von Femur 1 und Tibia 2 relativ zueinander verfolgt, entsprechende Positionsdatensätze können gespeichert werden.

Die Längsachse der Tibia 2 wird in geeigneter Weise in die Datenverarbeitungsanlage 17 eingegeben, dies kann beispielsweise in an sich bekannter Weise dadurch erfolgen, daß durch Bewegung des Beines um das Kniegelenk 3 und um den Knöchel die Lage der entsprechenden Gelenke bestimmt wird, eine Verbindungslinie dieser Gelenke entspricht dann der Längsachse der Tibia 2. In gleicher Weise kann die Längsachse des Femurs 1 in an sich bekannter Weise durch Bewegungen des Beins um das Hüftgelenk und das Knie bestimmt werden.

In der Datenverarbeitungsanlage wird daraufhin der Verlauf einer Ebene errechnet, die senkrecht auf der Längsachse der Tibia steht und die im Bereich des proximalen Endes der Tibia 2 verläuft, beispielsweise einige Millimeter unter dem höchsten Punkt des proximalen Tibiaendes. Eine derartige Ebene, wie sie beispielsweise durch die Schnittlinie 5-5 in Figur 4 angedeutet wird, wird nachfolgend als Projektionsebene 21 bezeichnet.

Die Datenverarbeitungsanlage projiziert in einem nächsten Schritt die Positionsdaten der Vorderkante 9 bei gestrecktem Bein auf die Projektionsebene 21 und erzeugt damit eine etwa U-förmige Projektionslinie 22, deren Verlauf von der Anordnung und vom Verlauf der Vorderkante 9 abhängt. Die Projektionslinie 22 umschließt mit ihrem U-förmigen Verlauf einen Bereich 23 auf der Projektionsebene 21, der nachstehend als erlaubter Bereich bezeichnet wird.

Das Implantat, das das vordere Kreuzband ersetzen soll, ist vorzugsweise im Querschnitt kreisrund, beispielsweise kann der Durchmesser des Implantates bei 6 mm bis 10 mm liegen, vorzugsweise etwa bei 8 mm.

Der gewählte Durchmesser des Implantates wird der Datenverarbeitungsanlage über eine Tastatur eingegeben, und die Datenverarbeitungsanlage erzeugt daraufhin eine Kreisfläche 24 in der Projektionsebene 21, die die Lage des Austrittspunktes 11 markiert.

Die Projektionsebene 21 mit der Projektionslinie 22 und der Kreisfläche 24 wird von der Datenverarbeitungsanlage 17 auf dem Bildschirm 20 dargestellt, so daß der Operateur genau erkennen kann, ob die Kreisfläche 24 im erlaubten Bereich liegt oder diesen teilweise verläßt. Die Darstellung auf dem Bildschirm 20 entspricht der Darstellung der Figur 5. Durch geeignete Mittel, beispielsweise einen Joystick oder eine Maus, kann die Kreisfläche 24 in der Projektionsebene 21 verschoben werden. Da das geschlossene Ende der Projektionslinie 22 zur Vorderseite des Knies hin weist und da der Austrittspunkt 11 zur Erhöhung der Stabilität möglichst weit anterior angeordnet sein soll, wird der Operateur die Kreisfläche 24 möglichst dicht an das anteriore Ende der Projektionslinie 22 verschieben, dabei aber genau darauf achten, daß die gesamte Kreisfläche 24, die dem Querschnitt des Implantats entspricht, innerhalb des erlaubten Bereiches bleibt und die Projektionslinie nicht schneidet. Wenn in dieser Weise eine optimale Lage der Kreisfläche 24 bestimmt ist, kann der Mittelpunkt dieser Kreisfläche 24 bestimmt werden. Dieser Mittelpunkt entspricht dem Austrittspunkt 11. Damit hat der Operateur einen Punkt ermittelt, an dem er das Implantat mit der Tibia 2 verbinden kann, beispielsweise dadurch, daß ein mit der Kreisfläche 24 übereinstimmendes Bohrloch gesetzt wird, in welches das Implantat eingezogen und innerhalb der Bohrung festgelegt wird. Es ist dann sichergestellt, daß das Implantat in einem Bereich der Tibiaoberfläche aus der Tibia 2 austritt, der eine freie Bewegung des Implantates ohne Berührung mit der knöchernen Struktur des Femurs ermöglicht, wenn Femur und Tibia gegeneinander bewegt werden.

Die Positionierung der Kreisfläche 24 im erlaubten Bereich kann von der Datenverarbeitungsanlage automatisch ausgeführt werden, es ist aber vorteilhaft, wenn der Operateur hier eine Wahlmöglichkeit hat, da er möglicherweise auch andere Optimierungskriterien verwenden kann, um einerseits einen optimalen Austrittspunkt 11 zu bestimmen und andererseits sicherzustellen, daß Kontakte des Implantates mit dem Femur zwischen Austrittspunkt 11 und Punkt 10 vermieden werden.

## Patentansprüche

1. Vorrichtung zur Bestimmung der Lage des Austrittspunktes (11) eines das vordere Kreuzbandes eines Kniegelenkes (3) ersetzenden Implantates an der Oberseite der Tibia (2) mit einem Navigationssystem (15), mit fest mit dem Femur (1) und der Tibia (2) verbundenen Markierelementen (13, 14), mit einem navigierten Taster (18) und mit einer einen Speicher und eine Recheneinheit aufweisenden Datenverarbeitungsanlage (17), der die durch das Navigationssystem (15) aufgenommenen Positionsdaten von mehreren Punkten längs der Vorderkante (9) der Notch (5) zuführbar und in den Speicher ablegbar sind, **dadurch gekennzeichnet, daß** die Recheneinheit so programmiert ist, daß sie die in dem Speicher abgelegten Positionsdaten und damit den Verlauf der Vorderkante (9) der Notch (6) bei gestrecktem Bein auf eine Projektionsebene (21) im Bereich der Tibiaoberseite projiziert, die senkrecht auf der Längsachse der Tibia (2) steht, und damit eine U-förmige projizierte Kurve (22) erzeugt, die einen Bereich umschließt, in dem sich der Austrittspunkt (11) befindet.

2. Vorrichtung nach Anspruch 1, **dadurch gekennzeichnet, daß** die Recheneinheit so programmiert ist, daß sie eine Kreisfläche (24), deren Durchmesser dem Durchmesser des verwendeten Implantates entspricht, in die Projektionsebene (21) projiziert, diese in der von der projizierten Kurve (22) umgebenen Fläche (23) so verschiebt, daß die Kreisfläche (24) die projizierte Kurve (22) nicht schneidet, und daß die Recheneinheit in einer der so erreichten Positionen der Kreisfläche (24) deren Mittelpunkt als Austrittspunkt (11) des Implantates wählt.

3. Vorrichtung nach Anspruch 2, **dadurch gekennzeichnet, daß** die Recheneinheit.so programmiert ist, daß sie eine Lage des Austrittspunktes (11) wählt, bei der dieser möglichst weit im vorderen Teil des von der projizierten Kurve (22) umgebenen Bereichs (23) und möglichst nahe an der projizierten Kurve (22) liegt.

4. Vorrichtung nach einem der Ansprüche 1 bis 3, **dadurch gekennzeichnet, daß** die Recheneinheit der Datenverarbeitungsanlage so programmiert ist, daß sie bei der Erfassung der Position von Punkten auf der Vorderkante (9) der Notch (6) auf einer Bildanzeige (20) der Datenverarbeitungsanlage (17) die Vorderkante (9) abbildet und dort die Punkte markiert, deren Positionsdaten zu erfassen sind.

5. Vorrichtung nach einem der Ansprüche 1 bis 4, **dadurch gekennzeichnet, daß** die Recheneinheit so programmiert ist, daß sie die projizierte Kurve (22) und den von ihr umgebenen Bereich (23) auf einer Bildanzeige (20) darstellt und auf dieser Bildanzeige (20) weiterhin ein verschiebbares Bild eines Anzeigeelementes (24) für die ausgewählte Position des Austrittspunktes (11) darstellt und die entsprechenden Positionsdaten der ausgewählten Position in einem Speicher ablegt.

6. Vorrichtung nach Anspruch 5, **dadurch gekennzeichnet, daß** das Anzeigeelement (24) eine Kreisfläche ist, deren Durchmesser dem des verwendeten Implantates entspricht.

## Claims

1. Apparatus for determining the position on the upper side of the tibia (2) of the exit point (11) of an implant replacing the anterior cruciate ligament of a knee joint (3), having a navigation system (15), having marking elements (13, 14) fixedly connected to the femur (1) and the tibia (2), having a navigated sensor (18) and having a data-processing system (17) which has a memory and a computer unit and to which the positional data for a number of points along the front edge (9) of the notch (5) recorded by the navigation system (15) can be fed and stored in the memory, **characterized in that** the computer unit is programmed in such a way that it projects the positional data stored in the memory, and with it the course of the front edge (9) of the notch (6) with the leg straight, onto a projection plane (21) in the region of the upper side of the tibia, which plane is perpendicular to the longitudinal axis of the tibia (2), and thereby produces a U-shaped projected curve (22) which encloses a region in which the exit point (11) is located.

2. Apparatus according to Claim 1, **characterized in that** the computer unit is programmed in such a way that it projects a circular area (24) of a diameter corresponding to the diameter of the implant used into the projection plane (21) and displaces it in the area (23) enclosed by the projected curve (22) such that the circular area (24) does not intersect the projected curve (22), and **in that** in one of the positions of the circular area (24) achieved in this way the computer unit selects its center point as the exit point (11) for the implant.

3. Apparatus according to Claim 2, **characterized in that** the computer unit is programmed in such a way that it selects a position for the exit point (11) in which the latter lies as far as possible in the front part of the region (23) enclosed by the projected curve (22) and as close as possible to the projected curve (22).

4. Apparatus according to one of Claims 1 to 3, **characterized in that** the computer unit of the data-processing system is programmed in such a way that, during the detection of the position of points on the front edge (9) of the notch (6), it forms an image of the front edge (9) on an image display (20) of the data-processing system (17) and marks there the points for which the positional data are to be recorded.

5. Apparatus according to one of Claims 1 to 4, **characterized in that** the computer unit is programmed in such a way that it presents the projected curve (22) and the region (23) enclosed by it on an image display (20) and also presents a displaceable image of a display element (24) for the selected position of the exit point (11) on this image display (20) and stores the corresponding positional data for the selected position in a memory.

6. Apparatus according to Claim 5, **characterized in that** the display element (24) is a circular area of a diameter corresponding to that of the implant used.

## Revendications

1. Dispositif pour déterminer la position du point de sortie (11) d'un implant qui remplace le ligament croisé antérieur d'une articulation du genou (3) sur la face supérieure du tibia (2) avec un système de navigation (15), avec des éléments marqueurs (13, 14) fixement reliés au fémur (1) et au tibia (2), avec un palpeur de navigation (18) et avec un dispositif de traitement des données (17) comportant une mémoire et une unité de calcul vers laquelle des données enregistrées par le système de navigation (15) concernant la position de plusieurs points le long du bord antérieur (9) du Notch (5) peuvent être acheminées et sauvegardées dans la mémoire, **caractérisé en ce que** l'unité de calcul est programmée de façon à projeter les données de position qui sont sauvegardées dans la mémoire et de ce fait la courbe du bord antérieur (9) du Notch (6) lorsque la jambe est tendue sur un plan de projection (21) dans la région de la face supérieure du tibia qui est perpendiculaire à l'axe longitudinal du tibia (2) et à créer de ce fait une courbe de projection en forme de U (22) qui entoure une région dans laquelle se trouve le point de sortie (11).

2. Dispositif selon la revendication 1, **caractérisé en ce que** l'unité de calcul est programmée de façon à projeter dans le plan de projection (21) une surface circulaire (24) dont le diamètre correspond au diamètre de l'implant utilisé, à la déplacer dans la surface (23) entourée par la courbe projetée (22) de façon à ce que la surface circulaire (24) ne recoupe pas la courbe projetée (22) et **en ce que** l'unité de calcul sélectionne dans la position ainsi obtenue de la surface circulaire (24) le point central de cette dernière en tant que point de sortie (11) de l'implant.

3. Dispositif selon la revendication 2, **caractérisé en ce que** l'unité de calcul est programmée de façon à sélectionner une position du point de sortie (11) dans laquelle celui-ci se situe le plus loin possible dans la partie antérieure de la région (23) entourée par la courbe projetée (22) et le plus près possible de la courbe projetée (22).

4. Dispositif selon l'une quelconque des revendications 1 à 3, **caractérisé en ce que** l'unité de calcul du dispositif de traitement des données (17) est programmée de façon à reproduire le bord antérieur (9) sur un afficheur vidéo (20), lors de l'enregistrement de la position des points sur le bord antérieur (9) du Notch (6) et à y marquer les points dont les données de position doivent être enregistrées.

5. Dispositif selon l'une quelconque des revendications 1 à 4, **caractérisé en ce que** l'unité de calcul est programmée de façon à représenter la courbe projetée (22) et la région (23) entourée par cette dernière sur un afficheur vidéo (20) et à représenter par ailleurs sur cet afficheur vidéo (20) une image déplaçable d'un élément d'affichage (24) pour la position sélectionnée du point de sortie (11) et à sauvegarder dans une mémoire les données de position correspondant à la position sélectionnée.

6. Dispositif selon la revendication 5, **caractérisé en ce que** l'élément d'affichage (24) est une surface circulaire, dont le diamètre correspond à celui de l'implant utilisé.
